# EUROPÄISCHE PATENTSCHRIFT

(11) **EP 0 534 166 B1**
(45) Veröffentlichungstag und Bekanntmachung des Hinweises auf die Patenterteilung: **30.07.1997**
(21) Anmeldenummer: 92114792.2
(22) Anmeldetag: 28.08.1992
(51) Int. Cl.: G01N 21/21

(54) **Verfahren und Vorrichtung zur quantitativen Bestimmung optisch aktiver Substanzen**
Procedure and apparatus for the quantitative determination of optic active substances
Procédé et dispositif pour la détermination quantitatif des substances optiquement actif

(30) Priorität: 28.08.1991 DE 4128458
(43) Veröffentlichungstag der Anmeldung: 31.03.1993
(73) Patentinhaber: Siemens-Elema AB, 171 95 Solna 1 (SE); SIEMENS AKTIENGESELLSCHAFT, 80333 München (DE)
(72) Erfinder: Schmidtke, Gerhard, Dr., W-7800 Freiburg (DE); Riedel, Wolfgang, W-7844 Neuenburg (DE); Wolf, Helmut, W-7802 Merzhausen (DE)

(56) Entgegenhaltungen:
- AT-B- 387 858
- DE-A- 2 513 937
- DE-C- 3 908 114
- US-A- 4 699 514
- US-A- 4 988 199

## Beschreibung

Die Erfindung betrifft ein Verfahren zur quantitativen Bestimmung der Konzentration optisch aktiver Substanzen, insbesondere von Glukose in der Körperflüssigkeit eines Patienten, durch Polarimetrie unter Erzeugung eines Vergleichssignals, insbesondere eines Differenz- oder Quotientensignals, mit zwei Lichtquellen, mit denen linear polarisierte Lichtbündel erzeugt werden, die die zu analysierende Substanz durchstrahlen und über einen Analysator einen Detektor beaufschlagen, wobei als feste Polarisationsrichtung des zweiten linear polarisierten Lichtbündels eine Polarisationsrichtung gewählt wird, die um einen vorbestimmten Winkel von der festen Polarisationsrichtung des ersten linear polarisierten Lichtbündels abweicht, daß die erste und zweite Lichtquelle im Wechsel mit einer Umschaltfrequenz ein- und ausgeschaltet werden und daß die von dem Detektor erzeugten zu den ersten und zweiten linear polarisierten Lichtbündeln gehörigen Meßsignale zur Erzeugung eines oder mehrerer Vergleichssignale, insbesondere von Differenz- oder Quotientensignalen, herangezogen werden, sowie betrifft auch eine Vorrichtung zur Durchführung des Verfahrens.

Ein solches Verfahren ist aus der DE-PS 39 08 114 C1 bekannt, wobei die dort beschriebene Vorrichtung es gestattet, die Konzentration optisch aktiver Substanzen quantitativ zu erfassen und gleichzeitig den Einfluß von Störungen, z.B. durch Alterung der sie bildenden Komponenten, zu vermindern. Diese Vorrichtung weist jedoch den Nachteil auf, daß mit ihr nur die Summe der optischen Drehungen der im Lichtweg befindlichen optisch aktiven Substanzen erfaßbar ist. Daher ist es nicht möglich, beim Auftreten mehrerer optisch drehender Substanzen die jeweiligen Einzelkonzentrationen interessierender Komponenten zu ermitteln.

Ausgehend von diesem Stand der Technik liegt der Erfindung die Aufgabe zugrunde, eine Vorrichtung der eingangs genannten Art anzugeben, die es gestattet, die Konzentration einer vorbestimmten optisch drehenden Substanz im Beisein anderer optisch drehender Stoffe quantitativ zu erfassen.

Diese Aufgabe wird erfindungsgemäß für ein Verfahren dadurch gelöst, daß durch ein dispergierendes Element im Strahlengang wellenlängenabhängige Absorption anzeigende Meßsignale auf einem spektral auflösenden Vielfachdetektor erzeugt werden, wobei deren jeweilige Extinktion im Bezug auf eine Referenzflüssigkeit auf die Konzentration von jeweils einer vorbestimmten, optisch drehenden Substanz in der Flüssigkeit schließen läßt, so daß das besagte ermittelte Vergleichssignal der optischen Drehung im Bezug auf diese vorbekannten optisch drehenden Substanzen korrigiert wird.

Diese Aufgabe wird erfindungsgemäß für eine Vorrichtung dadurch gelöst, daß mindestens ein dispergierendes Element im Strahlengang der Lichtbündel vorgesehen ist, daß die besagten oder weitere Detektoren als Zeilendetektoren in der Ebene des Brech- oder Beugungswinkels des dispergierenden Elementes ausgebildet sind, deren Photosignalausgänge an die Meßsignale zwischenspeichernde Steuer- und Auswerteelektronik angeschlossen ist, mit der die Spektralsignaturen der beiden Flüssigkeiten jeweils erfaßbar sind, aus denen die Konzentrationen von optisch drehenden Störsubstanzen ermittelbar sind, die einer in der Steuer- und Auswerteelektronik gespeicherten, von der Konzentration abhängigen optischen Drehung der vorbestimmten Substanzen entsprechen, und mit deren Rechenschaltung ein korrigiertes Regelsignal durch Summen- und/oder Differenzenbildung aus dem besagten Regelsignal mit den derart ermittelten Beträgen der optischen Drehung von einer oder mehreren vorbestimmten optisch drehenden Substanzen erzeugbar ist.

Dadurch, daß der Meß- und Referenzstrahl beispielsweise mit Hilfe einer prismenartig aufgebauten Küvette spektral zerlegt wird, ist es möglich, die optischen Drehungen für verschiedene in den Flüssigkeiten enthaltene optisch drehende Substanzen getrennt zu ermitteln und so das z.B. für eine Insulinpumpe zu verwendende Regelsignal in Abhängigkeit von der oder den gewünschten Substanzen zu ermitteln. Dabei ergibt sich der Vorteil, daß durch die spektrale Zerlegung der Meß- und Referenzlichtbündel Korrektursignale für vorbekannte, wahrscheinlich oder sicher in dem Dialysat enthaltene Störsubstanzen ermittelbar sind. Diese Korrektursignale werden zu dem Vergleichs- oder Regelsignal der optischen Drehung aller in der Flüssigkeit enthaltenen Stoffe je nach Richtung der optischen Drehung hinzugefügt oder abgezogen, so daß nur die von dem gesuchten Stoff erzeugte optische Drehung verbleibt, aus der das korrigierte Regelsignal hergeleitet werden kann. Damit ist der Einfluß von ebenfalls in der oder den Flüssigkeiten vorhandenen, weiteren optisch drehenden Substanzen, die den Meßwert der optischen Drehung verfälschen könnten, auf das zu verwertende Meßsignal stark vermindert.

Nachfolgend werden beispielhaft Ausführungsbeispiele der Erfindung anhand der Zeichnungen näher erläutert. Es zeigen:
- Fig. 1: eine schematische Draufsicht einer Vorrichtung zur quantitativen Bestimmung optisch aktiver Substanzen gemäß einem Ausführungsbeispiel der Erfindung,
- Fig. 2: eine Seitenansicht der Vorrichtung nach Fig. 1, und
- Fig. 3: eine schematische Draufsicht einer Vorrichtung zur quantitativen Bestimmung optisch aktiver Substanzen gemäß einem anderen Ausführungsbeispiel der Erfindung.

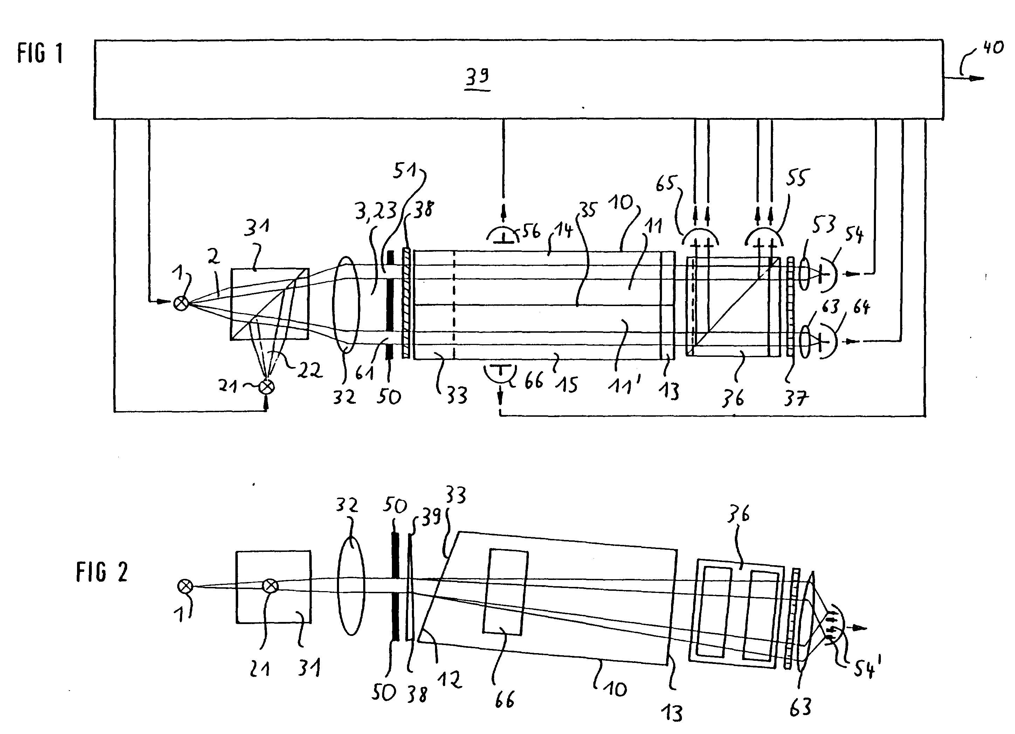

Die Fig. 1 zeigt in schematischer Darstellung eine Draufsicht auf eine Vorrichtung zur quantitativen Bestimmung optisch brechender, optisch absorbierender, optisch streuender und optisch aktiver Substanzen gemäß einem ersten Ausführungsbeispiel. Die Vorrichtung weist eine erste breitbandige Lichtquelle 1 und zweite breitbandige Lichtquelle 21 auf, die Licht der im wesentlichen selben Wellenlängen emittieren. Diese Lichtquellen 1 und 21 können als Lumineszenzdioden ausgebildet werden, die im gleichen Wellenlängenbereich von ca. 400 bis 900 Nanometer arbeiten.

Abweichend von der in Fig. 1 zur Veranschaulichung benutzten Darstellung des gleichzeitigen Betriebs der Lichtquellen 1 und 21, arbeiten diese im Meßbetrieb intermittierend.

Die Lichtquellen 1 und 21 erzeugen divergente Lichtstrahlen 2 und 22, die nach Durchgang durch einen polarisierenden Strahlteiler 31 von einer Kollimatorlinse 32 in einen parallelen Lichtstrahl umgelenkt werden. Dabei könnten anstelle des Strahteilerwürfels 31 mit einer Kollimatorlinse 32 auch ein teildurchlässiger Spiegel mit jeweils mindestens einer Linse für jeden Strahlengang und mit je einem Polarisator, z.B. in Gestalt einer Folie, verwendet werden.

Die entsprechenden Lichtstrahlen 3 und 23 treten durch Öffnungen 51 und 61 eines Strahlbegrenzers 50 hindurch. Anschließend durchstrahlen sie eine Küvette 10, die eine für die Wellenlängen des emittierten Lichtes der beiden Lichtquellen 1 und 21 transparente Vorderwand 12 und transparente Rückwand 13 aufweist. Die Küvette verfügt über zwei Kammern 14 und 15, die durch eine Trennwand 35 von einander getrennt sind. Diese Kammern 14 und 15 verlaufen parallel zu den einfallenden Lichtbündeln 3 und 23 und erstrecken sich von der Vorderwand 12 bis zur Rückwand 13. Die erste Kammer 14 ist mit einem Dialysat 11 gefüllt, wohingegen die zweite Kammer 15 mit einer Referenzflüssigkeit 11' gefüllt ist. Die vorzugsweise in den beiden Kammern 14 und 15 vorgesehenen Bohrungen zur Durchströmung mit den Flüssigkeiten 11 und 11' sind in den Figuren nicht dargestellt.

Die Doppelkammerküvette 10 weist als Vorderwand 12 vorteilhafterweise eine abgeschrägte Fläche 33 auf, die aus der Küvette einen Prismenkörper bildet. Vorteilhafterweise kann zwischen der Küvette 10 und dem Strahlbegrenzer 50 ein zusätzlicher Keil 38 zur angepaßten Strahlführung und zur verstärkenden spektralen Trennung der Lichtstrahlen 3 und 23 eingeführt werden.

Die durch diesen zusätzlichen Keil 38 spektral vorgetrennten Lichtbündel 3 und 23 fallen dann durch die abgeschrägte Prismenfläche 33 in die Küvette 10 ein.

Die Fläche 33 ist um einen Winkel aus der Normalebene der optischen Achse der Lichtbündel 3 und 23 verschwenkt, wobei dieser Winkel mit dem Brechwinkel 39 des Keils 38 in einer Ebene liegt. Die jeweils intermittierend die beiden Kammern 14 und 15 durchstrahlenden Lichtbündel 3 und 23 werden somit bei gleichzeitiger wellenlängenabhängiger Absorption spektral weiter aufgetrennt, bevor sie aus der Rückwand 13 der Küvette 10 heraustreten. In der Fig. 2 ist die Rückwand 13 der Küvette 10 im wesentlichen in der Normalebene zur durchschnittlichen optischen Achse der verschiedenen sich aufweitenden Lichtbündel 3 und 23 angeordnet. In einer anderen Fortbildung der Vorrichtung kann diese Rückwand 13 entsprechend der Vorderwand 10 ebenfalls abgeschrägt sein, so daß sich für das durch die Küvette 10 gebildete Prisma ein größerer Brechwinkel ergibt.

Durch den polarisierenden Strahlteiler 31, der zum Beispiel durch einen Strahlteilerwürfel gebildet werden kann, sind die Lichtbündel 3 und 23 der beiden Lichtquellen 1 und 21 senkrecht zueinander polarisiert. Hinter der Rückwand 13 der Doppelkammerküvette 10, wird als Analysator ein zweiter polarisierender Strahlteiler 36 verwendet, dessen Polarisationsebene gegenüber dem polarisierenden Strahlteiler 31 um 45° gedreht ist. Dadurch sind die Polarisationsrichtungen der Strahlen 3 und 23 der Lichtbündel von den Lichtquellen 1 und 21 symmetrisch, d.h. im Winkel von jeweils 45° zu den Durchlaßrichtungen des Analysators bezüglich beider Teilstrahlen angeordnet.

Das direkt durch den polarisierenden Strahlteiler 36 hindurchtretende Licht fällt über Sammellinsen 53 und 63 auf Zeilendetektoren 54 und 64. Der mit dem durch die Referenzflüssigkeit 11' hindurchgetretenen Licht beaufschlagte Zeilendetektor 64 bildet den ersten Referenzdetektor. Weiter bildet der Zeilendetektor 54, der mit dem durch das Dialysat 11 hindurchtretende Licht beaufschlagbar ist, einen ersten Meßdetektor. Die Zeilen der Detektoren 54 und 64 sind besser in der Fig. 2 zu erkennen, in welcher mit 54' die einzelnen Meßelemente der Zeilendetektoren 54 und 64 bezeichnet sind. Es ist aus der Fig. 2 leicht zu erkennen, daß die Zeilendetektoren 54 und 64 in der Ebene der Brechwinkel der Küvette 10 sowie des Keils 38 liegen. Die von den Zeilendetektoren 54 und 64 aufgenommenen Meßsignale werden der Auswerteschaltung 39, die mit einer Meßwertanzeige 40 versehen sein kann, zugeleitet. In dieser kann aufgrund der Lage der jeweiligen, Spektralausschnitte repräsentierenden Teilstrahlen auf den Zeilendetektoren 54 und 64 die optische Brechung sowie über die Intensitätsverhältnisse zwischen den einzelnen Detektoren 54' der Detektorzeilen 54 und 64 das Absorbtionsverhalten der verschiedenen Komponenten des Dialysats 11 bzw. der Referenzflüssigkeit 11' erfaßt werden.

Zur Erhöhung der spektralen Auflösung kann zwischen dem polarisierenden Strahlteiler 36 und den Sammellinsen 53 und 63 ein transparentes optisches Gitter 37 eingefügt werden. Es kann auch nur das optische Gitter 37 (ohne den Keil 38) vorgesehen sein und die Küvette 10 zudem parallele Wände 12 und 13 aufweisen. Dies führt unter Umständen zu einer niedrigeren Auflösung auf den Detektorzeilen 54 und 64, vermeidet aber Probleme in den optischen Elementen im Hinblick auf die nicht mehr parallele Strahlführung. Es muß nur mindestens ein dispergierendes Element im Lichtweg der Strahlenbündel 3 und 23 vorgesehen sein, so daß eine spektrale Unterscheidung auf Detektoren 54' und 55 sowie 65 möglich ist.

Die in der Fig. 1 eingezeichneten Sammellinsen 53 und 63 sind stark oval dargestellt, um die Dispersion in der Doppelkammerküvette 10 zu verdeutlichen. In der Praxis kann die Strahlführung jedoch so dimensioniert werden, daß kreisförmige Sammellinsen 53 und 63 verwendet werden können.

Weiterhin kann die optische Drehung der Einzelkomponenten aufgrund der verschiedenen, sich entsprechenden Signale der intermittiert arbeitenden Lichtquellen 1 bzw. 21 auf ein und demselben Einzeldetektor 54' des Zeilendetektors 54 erfaßt werden. Das Meßverfahren entspricht dem von der Anmelderin in der DE 39 08 114 C1 im Zusammenhang mit der dortigen Fig. 2 beschriebenen Meßverfahren, bei dem über die zwischengespeicherten Signalphotoströme ein Differenz- bzw. Quotientensignal gebildet wird, welche zur Bestimmung der Konzentration der optisch aktiven Substanz in dem Dialysat 11 benutzt werden kann.

Vorteilhafterweise wird aus dem spektral aufgelösten Lichtbündel durch die Intensitätsverhältnisse zwischen den einzelnen Detektoren 54' der Detektorzeilen 54 und 64 das Absorptionsverhalten von verschiedenen Komponenten des Dialysats 11 bzw. der Referenzflüssigkeit 11' erfaßt. Da das Meßsignal eines einem bestimmten Wellenlängenbereich zugeordneten Detektorelementes 54' einer vorbestimmten Substanz zuordbar ist, kann aus der Absorption der Lichtbündel direkt auf seine Konzentration geschlossen werden. Diese Konzentration einer vorher bekannten Störsubstanz, die natürlich derart ausgewählt werden, daß es wahrscheinlich ist, daß diese in dem Dialysat 11 auftreten, wird dann in der Auswerteschaltung 39 in eine dieser Substanz und Konzentration entsprechende optische Drehung umgerechnet, die in der wie unten angegebenen ermittelten optischen Gesamtdrehung enthalten ist. Die entsprechende Relation der Vorzeichen der beiden optischen Drehungen zueinander bestimmt, ob es sich bei dem optischen Drehwinkel durch die Störsubstanz um einen gleichgerichteten Anteil und somit um eine Subtraktion oder um einen entgegengesetzten Anteil und somit um eine Addition zu dem gemessenen Gesamtsignal optischer Drehung handelt.

Es ist bei einem z.B. hundert Einzeldetektoren 54' umfassenden Zeilendetektor 54 und 64 damit eine Erfassung der Störkonzentration von einer Vielzahl von bekannten, vorbestimmten, wahrscheinlich oder sicher in dem Dialysat 11 enthaltenen Substanzen möglich, wobei mit deren sich daraus ergebenden optischen Zusatz-Drehungen die ermittelte Gesamtdrehung des Dialysats 11 korrigiert wird, so daß im wesentlichen nur die optische Drehung z.B. der selbst nicht absorbierenden und damit spektral auflösbaren Glukose verbleibt.

Die von dem Strahlteiler 36 umgelenkten Lichtstrahlen der Lichtquellen 1 und 21 beaufschlagen positionsempfindliche Detektoren 55 und 65, die beispielsweise aus (in der Zeichnung nicht dargestellten) zwei halbkreisförmigen Detektorelementen bestehen können. Damit ist durch Vergleich der beiden Signale zueinander schon durch kleinste Abweichung die jeweilige Dispersion ermittelbar. Dieses Ergebnis ist genauer als die Ermittlung über die Zeilendetektoren 54 und 64, da dort eine Vielzahl von Detektoren 54' über einen großen Wellenlängenbereich empfindlich sind, wobei sich z.B. bei der langfristigen Implantation einer solchen Vorrichtung bei einem Patienten Unterschiede der Empfindlichkeit ergeben können, die nicht kompensierbar sind und das Meßergebnis verfälschen. Durch die ermittelte Dispersion ist die spektrale Messung der Zeilendetektoren 54 und 64 kalibrierbar.

Weiter ist durch Vergleich der Gesamtsignale der beiden Hälften des zweiten Meßdetektors 55 im Verhältnis zu dem Signal des zweiten Referenzdetektors 65 die gesamte optische Drehung des Dialysats 11 bestimmbar. Von dieser ausgehend werden dann, wie oben beschrieben, in der Rechenschaltung 39 die entsprechenden Korrekturdrehungen entsprechend den durch die Absorptionsmessungen ermittelten Konzentrationen an Störsubstanzen abgezogen, so daß sich schliesslich die Konzentration der z.B. Glukose ergibt. Solche oben erwähnten stark optisch drehenden Störsubstanzen können insbesondere Antibiotika sein, die in beide Richtungen drehend sein und deren Signalstärken jeweils 10 bis 50 Prozent des gemessenen Gesamtsignals ausmachen können.

Weiterhin sind seitlich an den Längsseiten der Kammern 14 und 15 der Küvette 10 Streulichtdetektoren 56 und 66 vorgesehen, mit deren Hilfe Streulichtmessungen vorgenommen werden können. Diese Streulichtmessungen verfolgen einen zweifachen Zweck. Das in die Küvette einströmende Dialysat enthält auch Eiweißmoleküle. Die größeren dieser Moleküle, insbesondere die über 5000 Atomeinheiten umfassen, werden üblicherweise bei der Erstellung des Dialysats durch z.B. Netze von dem Eintritt in die Küvette ferngehalten. Doch auch die Moleküle unter 5000 Atomeinheiten verursachen eine optische Drehung, die 10 Prozent des gewünschten Glukose-Signals der optischen Drehung ausmachen kann. Diese Moleküle streuen die die Küvette 10 durchlaufenden Lichtbündel.

Somit ist über die Intensität des Streulichts die Konzentration der Eiweißmoleküle erfaßbar. Die sich daraus ergebende, einen Störeinfluß bildende, optische Drehung der Eiweißmoleküle wird in der Rechenschaltung 39 von dem ermittelten Wert der optischen Drehung abgezogen. Dann verbleibt in dem erhaltenen Signal nur die optische Drehung der gesuchten Substanz, z.B. Glukose, nachdem nun die Störeinflüsse der optischen Drehung von nicht spektral identifizierbaren Stoffen wie Eiweißmolekülen durch die Streulichtmessungen und die von absorbierenden Substanzen über die Zeilendetektoren 54 und 64 aus dem mit den Detektoren 55 und 65 ermittelten Gesamtsignal der optischen Drehung herausgerechnet worden sind.

Zur Verbesserung der Meßempfindlichkeit wird vorteilhafterweise die in der DE 39 08 114 C1 beschriebene Lock-In-Technik verwandt, bei der z.B. die mit maximal einigen kHz intermittierend leuchtenden Lichtquellen 1 und 21 zusätzlich mit einer um mindestens eine Größenordnung höhere Modulationsfrequenz moduliert werden.

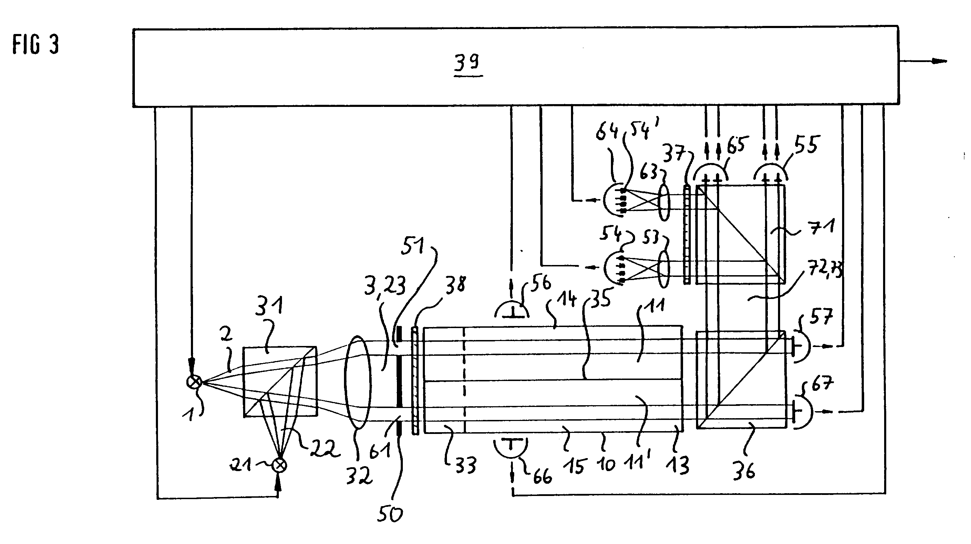

Die Fig. 3 zeigt eine schematische Draufsicht einer Vorrichtung zur quantitativen Bestimmung optisch aktiver Substanzen gemäß einem anderen Ausführungsbeispiel der Erfindung. Gleiche Merkmale tragen die gleichen, aus den Fig. 1 und 2 übernommenen Bezugszeichen.

Die Umlenkung durch einen direkt hinter der Küvette 10 angeordneten weiteren Strahlteilerwürfel 71 führt Lichtbündel 72 und 73 zu der schon im Zusammenhang mit der Fig. 1 beschriebenen Meßanordnung mit den Zeilendetektoren 54 und 64 zur Spektralanalyse und den Detektoren 55 und 65 zur Dispersionsbestimmung und zur Ermittlung des Gesamtsignals der optischen Drehung. Die direkt durch den Strahlteilerwürfel hindurchtretenden Strahlen beaufschlagen weitere Detektoren 57 und 67, wobei der Detektor 67 den Referenzdetektor bildet. Diese werden zur direkten Messung der gesamten optischen Drehung der in dem Dialysat 11 enthaltenen optisch drehenden Substanzen benutzt. Damit dienen die Messungen an den jeweils zwei sich halbkreisförmigen ergänzenden Detektoren 55 und 65 der redundanten Bestimmung dieser Signale und können zur Vereinfachung der Auswerteschaltung 39 weggelassen werden. Dann wird mit Hilfe der Detektoren 55 und 65 nur die Brechung gemessen, die der Zuordnung des Spektrums zu den Zeilendetektorelementen 54' dient. Allerdings ist der Einsatz der vier Detektoren-Anordnungen 54, 55, 56 und 57 (sowie 64, 65, 66 und 67 im Referenzkanal) zur teilweise redundanten Bestimmung der Meßwerte im Hinblick auf die gewünschte Langzeitstabilität bei der Implantation bei einem Menschen wünschenswert, da so häufige Operationen zum Austausch der Vorrichtungen vermieden werden können.

Schließlich können als Lichtquellen 1 und 21 auch Glühlampen verwendet werden, wobei dann zur Erzeugung der intermittierenden Strahlenbündel Zerhacker, auch Chopper genannt, eingesetzt werden.

## Patentansprüche

1. Verfahren zur quantitativen Bestimmung der Konzentration optisch aktiver Substanzen, insbesondere von Glukose in der Körperflüssigkeit eines Patienten, durch Polarimetrie unter Erzeugung eines Vergleichssignals, insbesondere eines Differenz- oder Quotientensignals, mit zwei Lichtquellen, mit denen lineare polarisierte Lichtbündel erzeugt werden, die die zu analysierende Substanz durchstrahlen und über einen Analysator einen Detektor beaufschlagen, wobei als feste Polarisationsrichtung des zweiten linear polarisierten Lichtbündels eine Polarisationsrichtung gewählt wird, die um einen vorbestimmten Winkel von der festen Polarisationsrichtung des ersten linear polarisierten Lichtbündels abweicht, daß die erste und zweite Lichtquelle im Wechsel mit einer Umschaltfrequenz ein- und ausgeschaltet werden und daß die von dem Detektor erzeugten zu den ersten und zweiten linear polarisierten Lichtbündeln gehörigen Meßsignale zur Erzeugung eines oder mehrerer Vergleichssignale, insbesondere von Differenz- oder Quotientensignalen, herangezogen werden, **dadurch gekennzeichnet,** daß durch ein dispergierendes Element im Strahlengang wellenlängenabhängige Absorption anzeigende Meßsignale auf einem spektral auflösenden Vielfachdetektor erzeugt werden, wobei deren jeweilige Extinktion im Bezug auf eine Referenzflüssigkeit auf die Konzentration von jeweils einer vorbestimmten, optisch drehenden Substanz in der Flüssigkeit schließen läßt, so daß das besagte ermittelte Vergleichssignal der optischen Drehung im Bezug auf diese vorbekannten optisch drehenden Substanzen korrigiert wird.

2. Verfahren nach Anspruch 1, dadurch gekennzeichnet, daß mit bezüglich dem Weg der Lichtbündel durch die zu analysierende Substanz seitlich angeordneten Streulichtdetektoren Streulichtsignale erfaßt werden, die in eine Konzentration von kleinen Makromolekülen umgerechnet werden, und daß das besagte ermittelte Vergleichssignal der optischen Drehung um die einer bestimmten Konzentration von Makromolekülen entsprechenden optische Drehung korrigiert wird.

3. Verfahren nach Anspruch 1 oder Anspruch 2, dadurch gekennzeichnet, daß die beiden Lichtbündel ebenfalls einen wenige Detektoren umfassenden Dispersionsdetektor beaufschlagen, mit dessen Ausgangssignal als Maß für die Dispersion die Vielfachdetektoren kalibriert werden.

4. Vorrichtung zur quantitativen Bestimmung der Konzentration optisch aktiver Substanzen (11), insbesondere von Glukose in der Körperflüssigkeit eines Patienten, durch Polarimetrie unter Erzeugung eines Vergleichssignals, insbesondere eines Differenz- oder Quotientensignals, mit zwei Lichtquellen (1, 21), mit denen linear polarisierte Lichtbündel (3, 23) von jeweils verschiedenen Polarisationsrichtungen erzeugbar (31) sind, die die Substanz (11) durchstrahlen, mit einer Zweikammer-Küvette (10; 14, 15) mit einer transparenten Vorderwand (12) sowie einer transparenten Rückwand (13), wobei die beiden Kammern (14, 15) sich parallel zueinander in Längsrichtung der Küvette (10) von deren Vorderwand (12) bis zu deren Rückwand (13) erstrecken und der Strahlengang der Lichtquellen (1, 21) in Längsrichtung der Küvette (10) ausgerichtet ist, daß die erste Kammer (15) mit einer Referenzflüssigkeit (11') und die zweite Kammer (14) mit der flüssigen, optisch aktiven Substanz (11) füllbar ist, daß die Lichtquellen (1, 21) im Wechsel einer Umschaltfrequenz ein- und ausgeschaltet sind, daß die Lichtbündel (3, 23) der Lichtquellen (1, 21) auch die Referenzflüssigkeit (11') durchstrahlen, wobei die Lichtbündel (3, 23) nach dem Durchtritt durch die Referenzflüssigkeit (11') durch einen Analysator (36) auf einen ersten Detektor (65) abgebildet werden, dessen Photosignalausgang an eine Steuer- und Auswerteelektronik (39) angeschlossen ist, in der die Meßsignale zwischenspeicherbar sind und die eine Rechenschaltung (39) zur Bildung eines Vergleichssignals aufweist, das ein der Lichtausbeute der Lichtquellen zugeordnetes Regelsignal darstellt, das in eine Versorgungsschaltung zur Versorgung einer der Lichtquellen einspeisbar ist, und daß die Lichtbündel nach dem Durchtritt durch die Substanz (11) durch den Analysator (36) auf einen zweiten Detektor (55) abgebildet werden, dessen Photosignalausgang an die Steuer- und Auswerteelektronik (39) angeschlossen ist, in der die Meßsignale zwischenspeicherbar sind und die eine Rechenschaltung (39) zur Bildung eines Vergleichssignals, insbesondere eines Quotienten- oder Differenzsignals, aufweist, das ein der optischen Drehung und der Konzentration der Substanz (11) zugeordnetes Regelsignal darstellt, **dadurch gekennzeichnet** daß mindestens ein dispergierendes Element (10, 37, 38) im Strahlengang der Lichtbündel (3, 23, 72, 73) vorgesehen ist, daß die besagten oder weitere Detektoren (54, 64) als Zeilendetektoren (54') in der Ebene (39) des Brech- oder Beugungswinkels des dispergierendes Elementes (10, 37, 38) ausgebildet sind, deren Photosignalausgänge an die Meßsignale zwischenspeichernde Steuer- und Auswerteelektronik (39) angeschlossen ist, mit der die Spektralsignaturen der beiden Flüssigkeiten (11, 11') jeweils erfaßbar sind, aus denen die Konzentrationen von optisch drehenden Störsubstanzen ermittelbar sind, die einer in der Steuer- und Auswerteelektronik (39) gespeicherten, von der Konzentration abhängigen optischen Drehung der vorbestimmten Substanzen entsprechen, und mit deren Rechenschaltung (39) ein korrigiertes Regelsignal durch Summen- und Differenzenbildung aus dem besagten Regelsignal mit den derart ermittelten Beträgen der optischen Drehung von einer oder mehreren vorbestimmten optisch drehenden Substanzen erzeugbar ist.

5. Vorrichtung nach Anspruch 4, dadurch gekennzeichnet, daß die Küvette (10) als dispergierendes Element in Gestalt eines prismenartigen Hohlkörpers (33; 13) ausgebildet ist, wobei die Vorderwand (12, 33) der Küvette als brechende Ebene (33) in einem von der Normalebene bezüglich der optischen Achse der Lichtbündel abweichenden Winkel ausgerichtet ist.

6. Vorrichtung nach einem der Ansprüche 4 oder 5, dadurch gekennzeichnet, daß zwischen der Küvette (10) und den Zeilendetektoren (54,64) das mindestens eine dispergierende Element in Gestalt eines optischen Gitters (37) angeordnet ist.

7. Vorrichtung nach einem der Ansprüche 4 bis 6, dadurch gekennzeichnet, daß zwischen dem Polarisator (31) und der Küvette (10) ein vordispergierendes Element (38) angeordnet ist.

8. Vorrichtung nach einem der Ansprüche 4 bis 7, dadurch gekennzeichnet, daß in Verlängerung der Längsachse der Küvette (10) ein polarisierender Strahlteiler (36) angeordnet ist, daß die abgelenkten oder die durchgehenden polarisierten Lichtbündel nach dem Durchgang durch den Strahlteiler (36) auf einen zweiten Meßdetektor (55) bzw. auf einen zweiten Referenzdetektor (65) abgebildet werden, deren Ausgangssignale die Auswerteschaltung (39) beaufschlagen und mit denen das besagte unkorrigierte Regelsignal erzeugbar ist.

9. Vorrichtung nach Anspruch 8, dadurch gekennzeichnet, daß die zweiten Detektoren (55,65) als zwei sich zu einem Gesamtdetektor ergebenden Halbkreisdetektoren ausgestaltet sind, mit deren die Auswerteschaltung (39) beaufschlagenden Ausgangssignalen die Dispersion der Lichtbündel (3, 23) erfaßbar ist.

10. Vorrichtung nach einem der Ansprüche 8 bis 9, dadurch gekennzeichnet, daß in Verlängerung einer der beiden Richtungen des hinter der Küvette (10) angeordneten polarisierenden Strahlteilers (36, 71) ein weiterer Strahlteiler (36, 71) angeordnet ist, daß die ihn durchstrahlenden, abgelenkten oder durchgehenden polarisierten Lichtbündel auf einen dritten Meßdetektor (57) bzw. auf einen dritten Referenzdetektor (67) abgebildet werden, deren Ausgangssignale die Auswerteschaltung (39) beaufschlagen und mit denen das besagte unkorrigierte Regelsignal erzeugbar ist.

11. Vorrichtung nach einem der Ansprüche 4 bis 10, dadurch gekennzeichnet, daß seitlich der Küvette (10) Streulichtdetektoren (56, 66) angeordnet sind, deren Ausgangssignale die Auswerteschaltung (39) beaufschlagen und aus denen ein Korrektursignal für nicht absorbierende optisch drehende Substanzen für das besagte unkorrigierte Regelsignal erzeugbar ist.

12. Vorrichtung nach Anspruch 11, dadurch gekennzeichnet, daß in der Auswerteschaltung (39) ein Schwellwertgeber für das Korrektursignal vorgesehen ist, wobei bei überschreiten des Schwellwertes ein Warnsignal erzeugbar ist, das auf eine Verunreinigung der zu prüfenden Substanz mit stark streuenden Großmolekülen hinweist.

## Claims

1. Procedure for the quantitative determination of the concentration of optically active substances, in particular of glucose in the body fluid of a patient, by means of polarimetry while generating a comparison signal, in particular a difference or quotient signal, the procedure using two light sources, with which linear polarized bundles of light are generated which stream through the substance to be analyzed and are incident on a detector via an analyzer, the fixed polarization direction of the second linearly polarized bundle of light being selected to be a polarization direction which deviates by a predetermined angle from the fixed polarization direction of the first linearly polarized bundle of light, the first and second light source being switched on and off at a changeover frequency, and the measured signals which are generated by the detector and belong to the first and second linearly polarized bundles of light being used for the generation of one or more comparison signals, in particular of difference or quotient signals, characterized in that, by means of a dispersing element in the beam path, measured signals indicating wavelength-dependent absorption are generated on a spectrally resolving multiple detector, their respective extinction in relation to a reference fluid allowing conclusions to be drawn about the concentration of a predetermined, optically rotating substance in each case in the fluid, so that the said determined comparison signal of the optical rotation is corrected in relation to these preknown optically rotating substances.

2. Procedure according to Claim 1, characterized in that using scattered light detectors arranged laterally in relation to the path of the bundles of light through the substance to be analyzed, scattered light signals are detected, which are converted into a concentration of small macromolecules, and in that the said determined comparison signal of the optical rotation is corrected by the optical rotation corresponding to a specific concentration of macromolecules.

3. Procedure according to Claim 1 or Claim 2, characterized in that the two bundles of light are likewise incident on a dispersion detector comprising few detectors with whose output signal as a measure of the dispersion the multiple detectors are calibrated.

4. Apparatus for the quantitative determination of the concentration of optically active substances (11), in particular of glucose in the body fluid of a patient, by means of polarimetry by generating a comparison signal, in particular a difference or quotient signal, the device having two light sources (1, 21), with which it is possible to generate linearly polarized bundles of light (3, 23) of respectively different polarization directions (31) which stream through the substance (11), having a two-chamber cuvette (10; 14, 15) having a transparent front wall (12) and a transparent rear wall (13), the two chambers (14, 15) extending parallel to one another in the longitudinal direction of the cuvette (10), from its front wall (12) to its rear wall (13), and the beam path of the light sources (1, 21) being aligned in the longitudinal direction of the cuvette (10), the first chamber (15) being able to be filled with a reference fluid (11') and the second chamber (14) being able to be filled with the fluid, optically active substance (11), the light sources (1, 21) being alternately switched on and off at a changeover frequency, the light bundles (3, 23) of the light sources (1, 21) also streaming through the reference fluid (11'), the bundles of light (3, 23), after passing through the reference fluid (11'), being imaged by an analyzer (36) onto a first detector (65), whose photosignal output is connected to a control and evaluation electronic means (39) in which the measured signals can be intermediately stored and which has a computing circuit (39) to form a comparison signal which represents a control signal associated with the light yield of the light sources, said signal being able to be fed into a supply circuit for supplying one of the light sources, and the bundles of light, after passing through the substance (11), being imaged by the analyzer (36) onto a second detector (55), whose photosignal output is connected to the control and evaluation electronic means (39), in which the measured signals can be intermediately stored and which has a computing circuit (39) to form a comparison signal, in particular a quotient or differential signal, which represents a control signal associated with the optical rotation and the concentration of the substance (11), characterized in that at least one dispersing element (10, 37, 38) is provided in the beam path of the bundles of light (3, 23, 72, 73), in that the said or further detectors (54, 64) are designed as line detectors (54') in the plane (39) of the refraction or diffraction angle of the dispersing element (10, 37, 38), whose photosignal outputs are connected to the control and evaluation electronic means (39) which store the measured signals intermediately and with which the spectral signatures of the two fluids (11, 11') can respectively be registered, from which the concentrations of optically rotating interfering substances can be determined, which correspond to an optical rotation of the predetermined substances which is stored in the control and evaluation electronic means (39) and depends on the concentration, and with whose computing circuit (39) a corrected control signal can be generated by means of sum and difference formation from the said control signal using the magnitudes, determined in this way, of the optical rotation of one or more predetermined optically rotating substances.

5. Apparatus according to Claim 4, characterized in that the cuvette (10) is designed as a dispersing element in the form of a prism-like hollow body (33; 13), the front wall (12, 33) of the cuvette being aligned as a refractive plane (33) at an angle deviating from the normal plane with respect to the optical axis of the bundles of light.

6. Apparatus according to either of Claims 4 and 5, characterized in that the at least one dispersing element is arranged between the cuvette (10) and the line detectors (54, 64) in the form of an optical grating (37).

7. Apparatus according to one of Claims 4 to 6, characterized in that a pre-dispersing element (38) is arranged between the polarizer (31) and the cuvette (10).

8. Apparatus according to one of Claims 4 to 7, characterized in that a polarizing beam splitter (36) is arranged in extension of the longitudinal axis of the cuvette (10), in that the polarized bundles of light which are deflected or pass through, after passing through the beam splitter (36), are imaged onto a second measuring detector (55) or onto a second reference detector (65), whose output signals are applied to the evaluation circuit (39) and with which the said uncorrected control signal can be generated.

9. Apparatus according to Claim 8, characterized in that the second detectors (55, 65) are configured as two semicircular detectors which result in an overall detector, using whose output signals, which are applied to the evaluation circuit (39), the dispersion of the bundles of light (3, 23) can be detected.

10. Apparatus according to either of Claims 8 and 9, characterized in that a further beam splitter (36, 71) is arranged in extension of one of the two directions of the polarizing beam splitter (36, 71) which is arranged downstream of the cuvette (10), in that the polarized bundles of light which stream through it, are deflected by it or pass through it are imaged onto a third measuring detector (57) or onto a third reference detector (67), whose output signals are applied to the evaluation circuit (39) and with which the said uncorrected control signal can be generated.

11. Apparatus according to one of Claims 4 to 10, characterized in that scattered light detectors (56, 66), whose output signals are applied to the evaluation circuit (39) and from which a correction signal for non-absorbing optically rotating substances for the said uncorrected control signal can be generated are arranged to the side of the cuvette (10).

12. Apparatus according to Claim 11, characterized in that a threshold value transmitter for the correction signal is provided in the evaluation circuit (39), it being possible, if the threshold value is exceeded, for a warning signal to be generated which points to a contamination of the substance to be tested with strongly scattering large molecules.

## Revendications

1. Procédé de détermination quantitative de la concentration de substances optiquement actives, notamment de glucose dans le liquide corporel d'un patient, par polarimétrie en produisant un signal de comparaison, notamment un signal de différence ou de quotient, par deux sources lumineuses, par lesquelles on produit des faisceaux lumineux polarisés linéairement, qui traversent la substance à analyser et qui rencontrent un détecteur par l'intermédiaire d'un analyseur, dans lequel on choisit comme direction fixe de polarisation du deuxième faisceau lumineux polarisé linéairement une direction de polarisation, qui s'écarte de la direction fixe de polarisation du premier faisceau lumineux polarisé linéairement d'un angle prédéterminé, on branche et on débranche en alternance les première et seconde sources lumineuses à une fréquence de commutation et on utilise les signaux de mesure produits par le détecteur et associés aux premier et second faisceaux lumineux polarisés linéairement pour produire un ou plusieurs signaux de comparaison, notamment des signaux de différence ou de quotient, caractérisé en ce que l'on produit par un élément dispersif dans le trajet des faisceaux des signaux de mesure indiquant une absorption en fonction de la longueur d'onde sur un détecteur multiple à résolution spectrale, l'extinction de chacune de celle-ci par rapport à un liquide de référence permettant de conclure sur la concentration d'une substance prédéterminée provoquant une rotation optique dans le liquide, de sorte à corriger ledit signal de comparaison déterminé de la rotation optique en référence à ces substances connues au préalable et provoquant une rotation optique.

2. Procédé suivant la revendication 1, caractérisé en ce que l'on détecte des signaux de lumière diffusée, qui sont convertis en une concentration de petites macromolécules, par des détecteurs de lumière diffusée disposés sur le côté par rapport au chemin des faisceaux lumineux dans la substance à analyser et on corrige ledit signal de comparaison déterminé de la rotation optique par la rotation optique correspondant à une concentration déterminée de macro molécules.

3. Procédé suivant la revendication 1 ou 3, caractérisé en ce que les deux faisceaux lumineux rencontrent également un détecteur de dispersion, qui comprend quelques détecteurs et par le signal de sortie duquel on calibre les détecteurs multiples comme mesure de la dispersion.

4. Dispositif de détermination quantitative de la concentration de substance (11) optiquement active, notamment de glucose dans le liquide corporel d'un patient, par polarimétrie en produisant un signal de comparaison, notamment un signal de différence ou de quotient, comportant deux sources lumineuses (1, 21), par lesquelles les faisceaux lumineux (3, 23) polarisés de manière rectiligne et de directions de polarisation différentes peuvent être produits (31) ces faisceaux lumineux traversant la substance (11), comportant une cuvette (10 ; 14, 15) ayant une paroi avant transparente (12) ainsi qu'une paroi arrière transparente (13), les deux chambres (14, 15) s'étendant parallèlement l'une à l'autre dans la direction longitudinale de la cuvette (10) de sa paroi avant (12) à sa paroi arrière (13) et le chemin des faisceaux des sources lumineuses (1, 21) étant orienté dans la direction longitudinale de la cuvette (10), la première chambre (15) pouvant être remplie d'un liquide (11') de référence et la seconde chambre (14) pouvant être remplie de la substance (11) liquide optiquement active, les sources lumineuses (1, 21) peuvent être branchées et débranchées en alternance à une fréquence de commutation, les faisceaux lumineux (3, 23) des sources (1, 21) de lumière traversent également le liquide (11') de référence, les faisceaux lumineux (3, 23) étant projetés, après la traversée du liquide (11') de référence par un analyseur (36) sur un premier détecteur (65) dont la sortie de signal photo-électrique est connectée à un dispositif électronique (39) de commande et d'exploitation, dans lequel les signaux de mesure peuvent être mémorisés temporairement et qui comporte un circuit (39) de calcul pour la formation d'un signal de comparaison, qui représente un signal de régulation associé au rendement lumineux des sources lumineuses, lequel peut être envoyé à un circuit d'alimentation de l'une des sources lumineuses, et les faisceaux lumineux sont projetés, après la traversée de la substance (11) par l'analyseur (36) sur un second détecteur (55), dont la sortie de signal photo-électrique est connectée au dispositif (39) électronique de commande et d'exploitation, dans lequel les signaux de mesure peuvent être mémorisés temporairement et qui comporte un circuit (39) de calcul pour l'information d'un signal de comparaison, notamment d'un signal de quotient ou de différence, qui représente un signal de régulation associé à la rotation optique et à la concentration de la substance (11), caractérisé en ce qu'il est prévu au moins un élément dispersif (10, 37, 38) dans le chemin des faisceaux lumineux (3, 23, 72, 73), lesdits ou d'autres détecteurs (54, 64) sont sous forme de détecteurs (54') à ligne dans le plan (39) de l'angle de réfraction ou de diffraction de l'élément dispersif (10, 37, 38), dont les sorties de signal photo-électrique sont connectées au dispositif (39) électronique de commande et d'exploitation qui mémorise temporairement des signaux de mesure et par lequel les signatures spectrales des deux liquides (11, 11') peuvent être détectées, les concentrations de substances parasites provoquant une rotation optique, qui correspondent à une rotation optique des substances prédéterminées dépendant de la concentration et mémorisées dans le dispositif électronique (39) de commande et d'exploitation, étant déterminées à partir des signatures spectrales et par le circuit (39) de calcul duquel un signal de régulation corrigé peut être produit par formation de la somme et de la différence dudit signal de régulation et des valeurs ainsi déterminées de la rotation optique d'une ou de plusieurs substances prédéterminées provoquant une rotation optique.

5. Dispositif suivant la revendication 4, caractérisé en ce que la cuvette (10) est sous forme d'un corps creux (33 ; 13) prismatique, la paroi avant (12, 33) de la cuvette étant orientée en tant que plan réfringent (33) sous un angle s'écartant du plan normal par rapport à l'axe optique des faisceaux lumineux.

6. Dispositif suivant l'une des revendications 4 ou 5, caractérisé en ce que le au moins un élément dispersif est disposé sous forme d'un réseau optique (37) entre la cuvette (10) et les détecteurs (54, 64) à ligne.

7. Dispositif suivant l'une des revendications 4 à 6, caractérisé en ce qu'un élément (38) prédispersif est disposé entre le polarisateur (31) et la cuvette (10).

8. Dispositif suivant l'une des revendications 4 à 7, caractérisé en qu'un séparateur (36) polarisant de faisceau est disposé dans le prolongement de l'axe longitudinal de la cuvette (10), les faisceaux lumineux polarisés déviés ou les faisceaux lumineux polarisés traversants sont projetés après la traversée du séparateur (36) de faisceaux sur un second détecteur (55) de mesure ou sur un second détecteur (65) de référence, dont les signaux de sortie sont appliqués au circuit (39) d'exploitation et par lesquels ledit signal de régulation non corrigé peut être produit.

9. Dispositif suivant la revendication 8, caractérisé en ce que les seconds détecteurs (55, 65) sont formés de deux détecteurs semi-circulaires se réunissant en un détecteur global, par les signaux de sortie s'appliquant au circuit (39) d'exploitation desquels la dispersion des faisceaux lumineux (3, 23) peut être détectée.

10. Dispositif suivant l'une des revendications 8 à 9, caractérisé en ce qu'un autre séparateur (36, 71) de faisceaux est disposé dans le prolongement de l'une des deux directions du séparateur (36, 71) de faisceaux polarisants disposés derrière la cuvette (10), les faisceaux lumineux polarisés traversants et déviés ou passants sont rejetés sur un troisième détecteur (57) de mesure ou sur un troisième détecteur (67) de référence, dont les signaux de sortie sont appliqués au circuit (39) d'exploitation et à l'aide desquels ledit signal de régulation non corrigé peut être produit.

11. Dispositif suivant l'une des revendications 4 à 10, caractérisé en ce que des détecteurs (56, 66) de lumière diffusée, dont les signaux de sortie sont appliqués au circuit (39) d'exploitation et à partir desquels un signal de correction pour des substances non absorbantes et provoquant une rotation optique peut être produit pour le signal de régulation non corrigé, sont disposés latéralement par rapport à la cuvette (10).

12. Dispositif suivant la revendication 11, caractérisé en ce qu'il est prévu dans le circuit (39) d'exploitation un indicateur de seuil pour le signal de correction, un signal d'avertissement, indiquant que la substance à examiner est polluée par de grosses molécules à forte dispersion, pouvant être produit lorsque le seuil est dépassé.
